# EUROPÄISCHE PATENTSCHRIFT

(11) **EP 0 549 898 B1**
(45) Veröffentlichungstag und Bekanntmachung des Hinweises auf die Patenterteilung: **07.02.1996**
(21) Anmeldenummer: 92120258.6
(22) Anmeldetag: 27.11.1992
(51) Int. Cl.: C07D 309/06, C07C 29/149

(54) **Verfahren zur Herstellung von primären Alkoholen**
Method for the preparation of primaryl alcohols
Méthode pour la préparation d'alcools primaires

(30) Priorität: 13.12.1991 DE 4141220
(43) Veröffentlichungstag der Anmeldung: 07.07.1993
(73) Patentinhaber: BASF Aktiengesellschaft, D-67063 Ludwigshafen (DE)
(72) Erfinder: Fischer, Rolf, Dr., W-6900 Heidelberg (DE); Goetz, Norbert, Dr., W-6520 Worms 1 (DE); Kuekenhoehner, Thomas, Dr., W-6737 Boehl-Iggelheim (DE); Schnurr, Werner, Dr., W-6719 Herxheim (DE); Weyer, Hans-Juergen, Dr., W-6800 Mannheim 1 (DE)

(56) Entgegenhaltungen:
- WO-A-82/03854
- JOURNAL OF ORGANIC CHEMISTRY, Bd. 47, Nr. 6, 12. März 1982, Washington, DC, US, Seiten 1011 - 1018 N. ACTON, ET AL.: 'Preparation and oxidation of the bis(tetra-n-butyl ammonium) salt of 2,2'-(2,7-pyrendiyl) bisÄpropanedinitrileÜ dianion'
- JOURNAL OF ORGANIC CHEMISTRY, Bd. 47, Nr. 16, 30. Juli 1982, Washington, DC, US, Seiten 3090 - 3094 T. SHONO, ET AL.: 'New synthesis of cyclopropanes from 1,3-dicarbonyl compounds utilising electroreduction of 1,3-dimethanesulphonates"

## Beschreibung

Die vorliegende Erfindung betrifft ein Verfahren zur Herstellung von primären Alkoholen, wobei die OH-Funktion eine Hydroxymethylgruppe ist durch katalytische Hydrierung entsprechender geminaler Dicarbonsäureester.

Für die Herstellung von durch eine Hydroxymethylgruppe substituierten Verbindungen aus geminalen Dicarbonsäureestern sind eine Reihe von Reaktionssequenzen bekannt:
So ist bekannt, geminale Dicarbonsäureester durch alkalische Verseifung zur entsprechenden geminalen Dicarbonsäure, thermische Abspaltung einer Carboxylgruppe und Veresterung der verbleibenden Carboxylgruppe in Monocarbonsäureester umzuwandeln. Die Monocarbonester werden dann durch Hydrierung in die Hydroxymethylverbindung umgewandelt. Auf diesem Wege wurde z.B. 1,3,6,8-Tetrahydropyren-2,2,7,7-tetracarbonsäureethylester zu 2,7-Bishydroxymethyl-1,3,6,8-tetrahydropyren umgesetzt (J. Org. Chem., Band 47, Seiten 1011 bis 1018 (1982).

Weiterhin ist bekannt, aus geminalen Dicarbonsäureestern durch Erhitzen mit Gemischen aus Mineralsäuren und Carbonsäuren entsprechende Monocarbonsäuren zu erhalten (Arch. Pharm. 319, Seiten 29 bis 37 (1986)), die anschließend zu den gewünschten Hydroxymethylverbindungen hydriert werden können.

Ferner ist aus Tetrahedron Lett. 27, Seiten 2283 bis 2286 (1986) bekannt, geminale Dicarbonsäureester einstufig durch Erhitzen in polaren Lösungsmitteln wie Dimethylsulfoxid oder Dimethylformamid in Gegenwart von Alkalichloriden oder Cyaniden in Monocarbonsäureester umzuwandeln. Diese Monocarbonsäureester lassen sich zu entsprechenden Hydroxymethylverbindungen hydrieren.

Diese Methoden zur Herstellung von Hydroxymethylverbindungen aus geminalen Dicarbonsäureestern besitzen den Nachteil, daß zahlreiche Reaktionsstufen erforderlich sind und daß im ersten Fall Salzanfall auftritt. Für die dritte, zweistufige Synthese sind im ersten Schritt sehr lange Reaktionszeiten erforderlich.

Es bestand daher die Aufgabe, den zuvor genannten Nachteilen abzuhelfen und ein einfacheres, möglichst einstufiges Verfahren für die Umwandlung von geminalen Dicarbonsäureestern in entsprechende Hydroxymethylverbindungen zu entwickeln.

Demgemäß wurde ein neues und verbessertes Verfahren zur Herstellung von Hydroxymethylverbindungen der allgemeinen Formel I 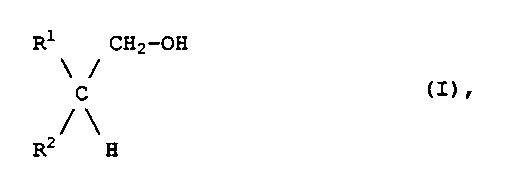
in der
- R¹,R²: Wasserstoff, C₁- bis C₁₂-Alkyl, C₃- bis C₈-Cycloalkyl, Acyl, Aryl oder C₇- bis C₂₀-Aralkyl oder gemeinsam -(CH₂)ₙ-X-(CH₂)ₘ-,
- R³: Wasserstoff oder C₁- bis C₁₂-Alkyl,
- X: Methylen, Sauerstoff, Schwefel, NH oder NR³
- n, m: 0 bis 8
bedeuten, gefunden, welches dadurch gekennzeichnet ist, daß man geminale Dicarbonsäuräeester der allgemeinen Formel II 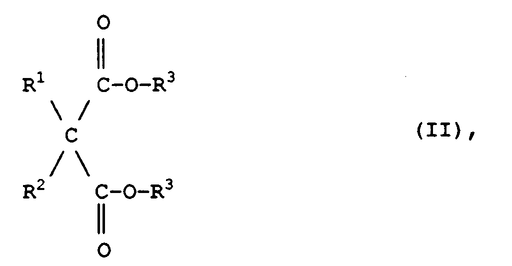
in der R¹, R² und R³ die obengenannte Bedeutung besitzen, bei Temperaturen von 50 bis 400°C und Drücken von 1 bis 400 bar in Gegenwart von Wasserstoff und Hydrierkatalysatoren umsetzt.

Es war überraschend, daß sich geminale Dicarbonsäureester der allgemeinen Formel II durch katalytische Hydrierung in einem Reaktionsschritt in entsprechende Hydroxymethylverbindungen der allgemeinen Formel I umsetzen lassen. Überraschend ist dies vor allem dadurch, daß bei der Hydrierung von geminalen Dicarbonsäureestern mit Lithiumalanat entsprechende geminale Dihydroxymethylverbindungen entstehen (EP-A-227 369, Seite 9; Journal of Organic Chemistry, Band 47, Seite 3091 (1982); Helvetica Chimica Acta, Band 66, 3, Seiten 891 bis 897 (1983)).

Das erfindungsgemäße Verfahren läßt sich wie folgt durchführen:
Die geminalen Dicarbonsäureester der allgemeinen Formel II können bei erhöhter Temperatur in Gegenwart von Hydrierkatalysatoren mit Wasserstoff in Hydroxymethylverbindungen der allgemeinen Formel I umgewandelt werden.

Die Umsetzung kann man diskontinuierlich oder kontinuierlich bei Temperaturen von 50 bis 400°C, vorzugsweise von 150 bis 350°C, insbesondere von 180 bis 250°C durchführen. Der Druck bei der Reaktion beträgt 1 bis 400 bar, insbesondere 50 bis 300 bar.

Die Umsetzung der geminalen Dicarbonsäurediester II zu Hydroxymethylverbindungen I kann in der Gasphase bei Temperaturen von 150 bis 250°C und Drücken von 50 bis 200 bar oder bevorzugt in der Flüssigphase bei Temperaturen von 180 bis 270°C und Drücken von 150 bis 400 bar erfolgen. Man arbeitet dabei vorteilhaft mit einer Katalysatorbelastung von 0,01 bis 5 g, insbesondere 0,1 bis 1g Dicarbonsäureester II pro g Katalysator und Stunde.

Die Reaktion kann in der Sumpf- oder Rieselfahrweise durchgeführt werden, wobei der Hydrierkatalysator in der Regel fest angeordnet wird. Es ist auch möglich, suspendierte Kaeinzusetzen. Als Reaktoren können z.B. Rohrreaktoren oder Rohrbündelreaktoren verwendet werden.

Die erfindungsgemäße Umsetzung kann in Abwesenheit von Lösungsmitteln durchgeführt werden. Es kann jedoch vorteilhaft sein, in Anwesenheit von Lösungsmitteln zu arbeiten. Als Lösungsmittel können beispielsweise Wasser, Ether wie Diethylether, Tetrahydrofuran und Dioxan, Aromaten wie Benzol, Toluol und die Xylole, chlorierte Kohlenwasserstoffe wie Chloroform und Methylenchlorid, Alkohole, z.B. C1- bis C8-Alkanole, bevorzugt C1- bis C5-Alkanole wie Methanol, Ethanol, n-Propanol, iso-Propanol, die Butanole und die Pentanole oder Gemische dieser Lösungsmittel verwendet werden. Als besonders vorteilhaft haben sich Alkohole erwiesen. Die Menge an Lösungsmittel kann in weiten Grenzen variiert werden und beträgt besonders bevorzugt, bezogen auf den eingesetzten geminalen Dicarbonsäurediester II, 5 bis 90 Gew.-%.

Die Hydrierkatalysatoren können allgemein übliche Katalysatoren sein, wie sie z.B. in H. Kropf, Methoden der organischen Chemie (Houben-Weyl), Band IV/1c, Georg Thieme Verlag Stuttgart, 1980, Seiten 16-26 und 26ff, beschrieben sind. So kommen als Hydrierkatalysatoren Elemente der VI. bis VIII. Nebengruppe des Periodensystems der Elemente z.B. in Form der Metalle, ihrer Oxide und Sulfide in Frage. Sie können z.B. als Trägerkatalysatoren, Skelettkatalysatoren, Schwarzkatalysatoren oder metallische Mischkatalysatoren eingesetzt werden. Beispiele sind Pt-Schwarz, Pt/C, Pt/Al₂O₃, PtO₂, Pd-Schwarz, Pd/C, Pd/Al₂O₃, Pd/SiO₂, Pd/CaCO₃, Pd/BaSO₄, Rh/C, Rh/Al₂O₃, Ru/C, Ru/SiO₂, Ni/SiO₂, Raney-Nickel, Co/SiO₂, Co/Al₂O₃, Raney-Kobalt, Fe, Fe-haltige Mischkatalysatoren, Re-Schwarz, Raney-Rhenium, Cu/SiO₂, Raney-Kupfer, PtO₂/Rh₂O₃, Pt/Pd/C, CuCr₂O₄, BaCr₂O₄, Ni/Cr₂O₃/Al₂O₃, Re₂O₇, Re₂S₇, CoS, NiS, MoS₃, Cu/SiO₂/Mgo, Ni/Cu/MoO₃/Al₂O₃.

Bevorzugte Hydrierkatalysatoren sind solche, die Kupfer und/oder eines oder mehrerer Metalle der VI., VII. und/oder VIII. Nebengruppe des Periodischen Systems der Elemente enthalten, insbesondere Nickel, Cobalt, Ruthenium, Rhenium, Kupfer, Chrom, Molybdän und/oder Mangan. Sie können sowohl als Trägerkatalysatoren oder in kompakter Form, d.h. ohne Träger eingesetzt werden. Als Trägermaterial können z.B. Siliciumdioxid, Aluminiumoxide, Titanoxide, Aktivkohle, Silikate oder Zeolithe verwendet werden.

Die Umsetzung der geminalen Dicarbonsäureester II in der Flüssigphase wird beispielsweise so durchgeführt, daß man ein Gemisch aus II und gegebenenfalls einem Lösungsmittel in Gegenwart eines suspendierten Hydrierkatalysators und Wasserstoff unter Druck auf die gewünschte Reaktionstemperatur erhitzt. Nach Ablauf der Reaktion wird das Reaktionsgemisch abgekühlt und entspannt und der Katalysator, z.B. durch Filtration entfernt. Das Reaktionsgemisch kann anschließend zur Gewinnung der gewünschten Hydroxymethylverbindung I z.B. fraktionierend destilliert werden.

Arbeitet man, was besonders bevorzugt ist, in Gegenwart eines als Festbett angeordneten Hydrierkatalysators in der Flüssigphase, so leitet man den geminalen Dicarbonsäureester II, gegebenenfalls zusammen mit einem Lösungsmittel, in Gegenwart von Wasserstoff unter Druck in Sumpf- oder Rieselfahrweise über den Hydrierkatalysator. Der Hydrieraustrag wird abgekühlt und entspannt und anschlieáend zur Gewinnung der Hydroxymethylverbindung I z.B. fraktionierend destilliert.

Das Zwischenglied X, die Substituenten R¹, R² und R³ und die Indizes n und m in den Verbindungen I und II haben folgende Bedeutungen:

### R¹, R²

- unabhängig voneinander
- Wasserstoff,
- C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Undecyl, n-Dedecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- Acyl wie Acetyl
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- -(CH₂)ₙ-X-(CH₂)ₘ,

### X

- Methylen (-CH₂-),
- Sauerstoff,
- Schwefel,
- NH,
- NR³,

### R³

- Wasserstoff,
- C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,

### n, m

- jeweils eine ganze Zahl von 0 bis 8 wie 0, 1, 2, 3, 4, 5, 6, 7 und 8, bevorzugt jeweils eine ganze Zahl von 1 bis 4 wie 1, 2, 3 und 4.

Geeignete geminale Dicarbonsäureester und ihre Derivate der Formel II sind z.B. in 2-Stellung substituierte Malonsäurediester wie 2-Methylmalonsäuredimethylester, 2-Ethylmalonsäurediethylester, 2-Acetylmalonsäuredimethylester, 2-Phenylmalonsäurediethylester, 2,2-Dimethylmalonsäuredimethylester, 2,2-Diacetylmalonsäuredipropylester, 2-Benzylmalonsäuredimethylester, 1,1-Cyclopropandicarbonsäuredimethylester, 1,1-Cyclohexandicarbonsäurediethylester, Tetrahydropyran-4,4-dicarbonsäuredimethylester, Tetrahydrothiopyran-4,4-dicarbonsäuredimethylester, 1,1-Cyclopentandicarbonsäuredimethylester.

Die für die erfindungsgemäße Umsetzung benötigten Dicarbonsäureester II sind z. B. durch Mono- oder Bisalkylierung oder Acylierung von Malonsäurediestern zugänglich.

Die nach dem erfindungsgemäßen Verfahren erhältlichen Mono-carbonsäureester stellen wertvolle, vielseitig für organische Synthese verwendbare Zwischenprodukte dar.

Zusammensetzung der in den Beispielen verwendeten Katalysatoren:

| Katalysator | Zusammensetzung |
|---|---|
| A | 64 % CoO, 18 % CuO, 7 % MnO₂, 4 % MoO₂, 0,2 % |
| | Na₂O |
| B | 65 % CuO, 12,2 % SiO₂, 6,2 % MgO |
| C | 50 % NiO, 17 % CuO, 2 % MoO₃, 31 % Al₂O₃ |

### Beispiel 1

Pro Stunde wurden durch einen Rohrreaktor, der 34 g des Katalysators A enthielt, in Rieselfahrweise 15 g einer 20 gew.-%igen Lösung von Tetrahydropyran-4,4-dicarbonsäuredimethylester in Methanol geleitet. Die Hydrierung wurde 5 Stunden lang bei 200°C und 200 bar Wasserstoff betrieben. Laut quantitativer Gaschromatographie betrug die Ausbeute am 4-Hydroxymethyltetrahydropyran während dieser Zeit im Mittel 91 % (bezogen auf eingesetzten Diester II).

### Beispiel 2

Wurde anstelle von Katalysator A unter sonst gleichen Bedingungen Katalysator B eingesetzt, so betrug die Ausbeute an 4-Hydroxymethyl-tetrahydropyran 84 % (bezogen auf eingesetzten Diester II).

### Beispiel 3

Pro Stunde wurden durch einen Rohrreaktor, der 17 g des Katalysator C enthielt, in Rieselfahrweise 20 ml einer 20 gew.-%igen Lösung von Tetrahydropyran-4,4-dicarbonsäuredimethylester in Methanol geleitet. Die Hydrierung wurde 4 Stunden lang bei 200°C und 200 bar betrieben. Laut quantitativer Gaschromatographie betrug die Ausbeute an 4-Hydroxymethyltetrahydropyran während dieser Zeit im Mittel 25 % (bezogen auf eingesetzten Tetrahydropyran-4,4-dicarbonsäureester), der Diester-Umsatz 37 %.

### Beispiel 4

Beispiel 1 wurde mit folgenden Änderungen wiederholt:
Anstelle der methanolischen Einsatzlösung wurde eine Lösung von 20 Gew.-% Diester in n-Butanol verwendet. Bei einem Umsatz von 100 % wurde eine Ausbeute an 4-Hydroxymethyltetrahydropyran von 91 % gefunden.

### Beispiel 5

Beispiel 1 wurde mit folgenden Änderungen wiederholt:
20 ml einer 20 %igen Lösung von Cyclohexan-1,1-dicarbonsäure- dimethylester in n-Butanol als Lösungsmittel wurden bei 220°C und 200 bar pro Stunde zugefahren. Die Hydrierung wurde drei Stunden lang bei 220°C und 200 bar Wasserstoff betrieben. Die durchschnittliche Ausbeute an Hydroxymethylcyclohexan betrug 90 %, der Umsatz war quantitativ.

### Beispiel 6

Beispiel 5 wurde mit folgenden Unterschieden wiederholt:
Pro Stunde wurden 20 ml einer 20 gew.-%igen Lösung von 2,2-Dimethylmalonsäuredimethylester in n-Butanol zugeführt. Über einen Zeitraum von 7 Stunden wurde durch quantitative gaschromatographische Analyse eine iso-Butanol-Ausbeute von 93 % beobachtet. Der Diester- Umsatz war vollständig.

### Beispiel 7

Beispiel 1 wurde mit dem Unterschied wiederholt, daß eine 20 gew.-%ige Lösung von Tetrahydropyran-4,4-dicarbonsäuredi-n-butylester in n-Butanol verwendet wurde. Die Hydrierung wurde 4 Stunden bei 220°C und 200 bar Wasserstoff betrieben. Laut quantitativer Gaschromatographie betrug die Ausbeute an 4-Hydroxymethyltetrahydropyran während dieser Zeit im Mittel 90 % (bezogen auf eingesetzten Diester II).

## Patentansprüche

1. Verfahren zur Herstellung von Hydroxymethylverbindungen der allgemeinen Formel I 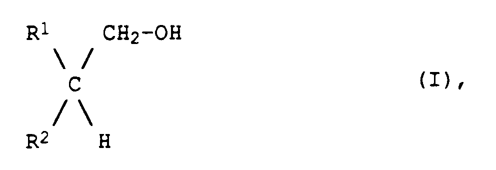 in der
R¹, R² Wasserstoff, C₁- bis C₁₂-Alkyl, C₃- bis C₈-Cycloalkyl, Acyl, Aryl oder C₇- bis C₂₀-Aralkyl oder gemeinsam -(CH₂)ₙ-X-(CH₂)ₘ-,
R³ Wasserstoff oder C₁- bis C₁₂-Alkyl,
X Methylen, Sauerstoff, Schwefel, NH oder NR³
n, m 0 bis 8
bedeuten, dadurch gekennzeichnet, daß man geminale Dicarbonsäureester der allgemeinen Formel II, 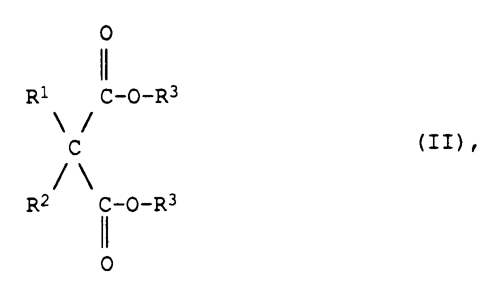 in der R¹, R² und R³ die obengenannte Bedeutung besitzen, bei Temperaturen von 50 bis 400°C und Drücken von 1 bis 400 bar in Gegenwart von Wasserstoff und Hydrierkatalysatoren umsetzt.

2. Verfahren nach Anspruch 1, dadurch gekennzeichnet, daß man Hydrierkatalysatoren verwendet, die Kupfer und/oder ein oder mehrere Metalle der Elemente der VII. und/oder VIII. Nebengruppe des Periodensystems der Elemente enthalten.

3. Verfahren nach Anspruch 1, dadurch gekennzeichnet, daß man in der Flüssigphase arbeitet.

## Claims

1. A process for preparing hydroxymethyl compounds of the general formula I 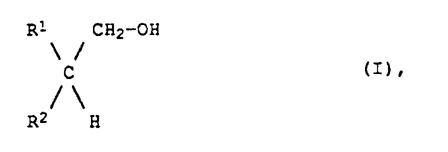 where
R¹ and R² are each hydrogen, C₁- to C₁₂-alkyl, C₃- to C₈-cycloalkyl, acyl, aryl or C₇- to C₂₀-aralkyl or together are -(CH₂)ₙ-X-(CH₂)ₘ-,
R³ is hydrogen or C₁- to C₁₂-alkyl,
X is methylene, oxygen, sulfur, NH or NR³,
n and m are each 0 to 8,
which comprises reacting geminal dicarboxylic esters of the general formula II 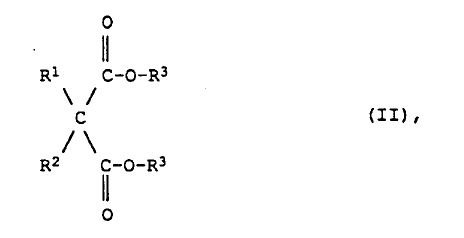 where R¹, R² and R³ have the abovementioned meanings, with hydrogen in the presence of hydrogenation catalysts at from 50 to 400°C and under from 1 to 400 bar.

2. A process as claimed in claim 1, wherein the hydrogenation catalysts contain copper and/or one or more metals of group VIIB and/or VIII of the periodic table.

3. A process as claimed in claim 1, which is carried out in the liquid phase.

## Revendications

1. Procédé de préparation de composés hydroxyméthyliques de la formule générale I 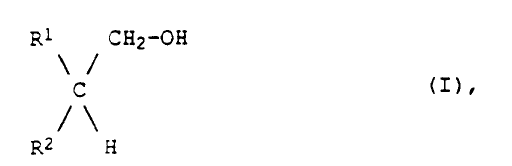 dans laquelle
R¹ et R² représentent chacun un atome d'hydrogène, un radical alkyle en C₁ à C₁₂, cycloalkyle en C₃ à C₈, acyle, aryle, ou aralkyle en C₇ à C₂₀, ou forment ensemble un radical -(CH₂)ₙ-X-(CH₂)ₘ-,
R³ représente un atome d'hydrogène ou un radical alkyle en C₁ à C₁₂,
X représente le radical méthylène, un atome d'oxygène, de soufre, un radical NH ou NR³,
n et m ont chacun une valeur qui varie de 0 à 8
caractérisé en ce que l'on fait réagir des esters d'acides dicarboxyliques géminaux de la formule générale II 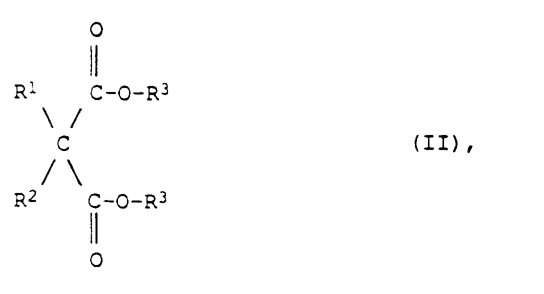 dans laquelle R¹, R² et R³ possèdent les significations qui leur ont été attribuées ci-dessus, à des températures de 50 à 400°C et sous des pressions de 1 à 400 bars, en présence d'hydrogène et de catalyseurs d'hydrogénation.

2. Procédé suivant la revendication 1, caractérisé en ce que l'on utilise des catalyseurs d'hydrogénation qui contiennent du cuivre et/ou un ou plusieurs métaux des éléments des septième et/ou huitième sous-groupes du système périodique des éléments.

3. Procédé suivant la revendication 1, caractérisé en ce que l'on travaille en phase liquide.
